(19) Europäisches Patentamt
European Patent Office
Office européen des brevets

(11) Publication number: 0 542 217 A2

(12) # EUROPEAN PATENT APPLICATION

(21) Application number: 92119267.0

(22) Date of filing: 11.11.92

(51) Int. Cl.5: C07C 31/20, C07C 29/141, C07D 309/22, C07C 45/60, C07C 47/12, C08G 63/12

(30) Priority: 12.11.91 US 790872

(43) Date of publication of application: 19.05.93 Bulletin 93/20

(84) Designated Contracting States: AT BE DE DK ES FR GB IT NL SE

(71) Applicant: UNION CARBIDE CHEMICALS & PLASTICS TECHNOLOGY CORPORATION
39 Old Ridgebury Road
Danbury, Connecticut 06817-0001(US)

(72) Inventor: Argyropoulos, John Nicholas
35 Michael Street
Scott Depot, West Virginia 25560(US)
Inventor: Keen, Brian Terry
1528 Autumn Road
Charleston, West Virginia 25314(US)
Inventor: Best, Donald Charles
411 Sheridan Circle
Charleston, West Virginia 25314(US)
Inventor: Koleske, Joseph Victor
1513 Brentwood Road
Charleston, West Virginia 25314(US)

(74) Representative: Brandes, Jürgen, Dr. rer. nat. et al
Wuesthoff & Wuesthoff, Patent- und Rechtsanwälte, Schweigerstrasse 2
W-8000 München 90 (DE)

(54) **Substituted 1,5-pentanediols and processes for the preparation thereof.**

(57) This invention relates to substituted 1,5-pentanediols and derivatives thereof and to processes for the preparation of substituted 1,5-pentanediols and derivatives thereof. The substituted 1,5-pentanediols and derivatives thereof are useful alone or in the production of intermediates, and have utility in coatings, adhesives, inks, fibers, pharmaceutical intermediates, sealants, stereolithography, as well as in other end uses.

EP 0 542 217 A2

## Brief Summary of the Invention

### Technical Field

This invention relates to substituted 1,5-pentanediols and derivatives thereof and to processes for the preparation of substituted 1,5-pentanediols and derivatives thereof. The substituted 1,5-pentanediols and derivatives thereof are useful alone or in the production of intermediates, and have utility in coatings, adhesives, inks, fibers, pharmaceutical intermediates, sealants, stereolithography, as well as in other end uses.

### Background of the Invention

Diols in general are well known in industry and many are important articles of commerce. It is well known that one or more diols such as ethylene glycol, diethylene glycol, 1,4-butanediol, 2,2-dimethyl-1,3-propanediol, and 1,6-hexanediol are used as ingredients for the preparation of polyesters, as short chain extenders for polyurethanes, as antifreeze agents, as humectants, as emollients, as well as for other end uses. The hydrocarbon nature of diols is often lost in derivatives prepared therefrom and the products prepared from such derivatives take on the nature of the products so made, i.e., that of a polyester or a polyurethane or the like.

U.S. Patent No. 2,600,275 describes the preparation of a class of substituted 1,5-pentanediols in which the substitution is alkyl or substituted alkyl and ether substituted alkyl groups. Illustrative of these compounds is 2,4-dialkyl-2-alkoxymethyl-1,5-pentanediol.

U.S.S.R. 376,349, Chemical Abstracts 79, 41899z (1973), and Osnov. Organ. Sintex i Neftekhimiya, Leningrad, (11), 52-5 (1979), Chemical Abstracts 92, 215204e (1980), describe the preparation of 2,4-diethyl-3-propyl-1,5-pentanediol by hydrogenation of a mixture of 12-carbon lactones at 80-130 °C in an alcohol medium at a pressure of 100-300 atmospheres of hydrogen and in the presence of a copper-chromium catalyst. U.S.S.R. 584,018, Chemical Abstracts 88, 62910x (1978), describes unsaturated polyesters prepared from 2,4-diethyl-3-propyl-1,5-pentanediol, triols, maleic anhydride and modifying acids. It is stated that the unsaturated polyesters have improved solubility and compatibility with styrene.

U.S. Patent No. 4,141,850 discloses compositions of an aqueous solution containing sulfuric acid, hydrogen peroxide, and an effective amount of a primary diol. The primary diol is of the form:

$HOCH_2-R^1-CH_2OH$

wherein $R^1$ is selected from

a) $-(CR^2R^3)_n-$ where each $R^2$ and $R^3$ is independently from each other is either hydrogen or an alkyl group of one to four carbon atoms and n is at least 2, or

b) a substituted or unsubstituted cycloparaffinic group.

### Disclosure of the Invention

This invention relates to liquid hydrocarbon diols represented by the formula:

$HO - R' - OH$

wherein R' is a substituted hydrocarbon residue having 8 or more carbon atoms in which the primary hydroxyl groups are separated by 4 or more carbon atoms linearly arranged and in which at least one of said carbon atoms linearly arranged is a disubstituted carbon atom or at least 2 of said carbon atoms linearly arranged are monosubstituted carbon atoms, said liquid hydrocarbon diol existing as a liquid at a temperature of 35 °C or less, and to derivatives of said liquid hydrocarbon diols.

More particularly, this invention relates to liquid hydrocarbon diols represented by the formula:

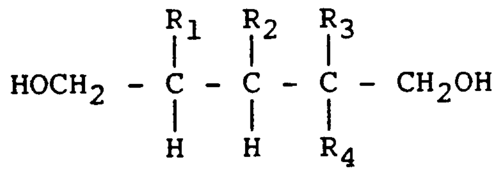

(I)

wherein $R_1$ is hydrogen or linear or branched alkyl having from 1 to 3 carbon atoms, and $R_2$, $R_3$ and $R_4$ are the same or different and are hydrogen or linear or branched alkyl having from 1 to 4 carbon atoms, said liquid hydrocarbon diol existing as a liquid at a temperature of 35 °C or less; provided (a) at least 2 of $R_1$, $R_2$, $R_3$ and $R_4$ are other than hydrogen; (b) the total number of carbon atoms in $R_1$, $R_2$, $R_3$ and $R_4$ together is 5 or more except as follows: (i) when $R_2$ is ethyl and one of $R_1$, $R_3$ and $R_4$ is methyl, then the remaining 2 of $R_1$, $R_3$ and $R_4$ can be hydrogen; (ii) when $R_3$ and $R_4$ are independently methyl and propyl, then $R_1$ and $R_2$ can be hydrogen; and (iii) when $R_1$ is methyl and $R_2$ is ethyl and one of $R_3$ and $R_4$ is methyl, then the remaining $R_3$ or $R_4$ can be hydrogen; (c) when the total number of carbon atoms in $R_1$ and $R_2$ is 4 or more, then the total number of carbon atoms in $R_2$ is 3 or less except when $R_1$ is propyl and $R_3$ and $R_4$ are hydrogen, then $R_2$ can be butyl; and (d) when $R_1$ is ethyl and $R_2$ is propyl, then $R_3$ is other than ethyl; and to derivatives of said liquid hydrocarbon diols.

This invention also relates to a process comprising:

(a) contacting a substituted vinyl ether with a substituted or unsubstituted acrolein to form a substituted 3,4-dihydropyran;

(b) contacting the substituted 3,4-dihydropyran with an acid catalyst to form a substituted dialdehyde; and

(c) hydrogenating the substituted dialdehyde in the presence of a catalyst to form a liquid hydrocarbon diol represented by the formula:

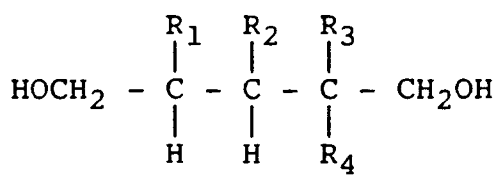

wherein $R_1$ is hydrogen or linear or branched alkyl having from 1 to 3 carbon atoms, and $R_2$, $R_3$ and $R_4$ are the same or different and are hydrogen or linear or branched alkyl having from 1 to 4 carbon atoms, said liquid hydrocarbon diol existing as a liquid at a temperature of 35 °C or less; provided (a) at least 2 of $R_1$, $R_2$, $R_3$ and $R_4$ are other than hydrogen; (b) the total number of carbon atoms in $R_1$, $R_2$, $R_3$ and $R_4$ together is 5 or more except as follows: (i) when $R_2$ is ethyl and one of $R_1$, $R_3$ and $R_4$ is methyl, then the remaining 2 of $R_1$, $R_3$ and $R_4$ can be hydrogen; (ii) when $R_3$ and $R_4$ are independently methyl and propyl, then $R_1$ and $R_2$ can be hydrogen; and (iii) when $R_1$ is methyl and $R_2$ is ethyl and one of $R_3$ and $R_4$ is methyl, then the remaining $R_3$ or $R_4$ can be hydrogen; (c) when the total number of carbon atoms in $R_1$ and $R_2$ is 4 or more, then the total number of carbon atoms in $R_2$ is 3 or less except when $R_1$ is propyl and $R_3$ and $R_4$ are hydrogen, then $R_2$ can be butyl; and (d) when $R_1$ is ethyl and $R_2$ is propyl, then $R_3$ is other than ethyl.

## Detailed Description

As indicated above, this invention relates to liquid hydrocarbon diols represented by the formula:

$$HO - R' - OH$$

wherein R' is a substituted hydrocarbon residue having 8 or more carbon atoms in which the primary hydroxyl groups are separated by 4 or more carbon atoms linearly arranged and in which at least one of said carbon atoms linearly arranged is a disubstituted carbon atom or at least 2 of said carbon atoms linearly arranged are monosubstituted carbon atoms, said liquid hydrocarbon diol existing as a liquid at a temperature of 35 °C or less, and to derivatives of said liquid hydrocarbon diols.

As also indicated above, this invention more particularly relates to liquid hydrocarbon diols represented by the formula:

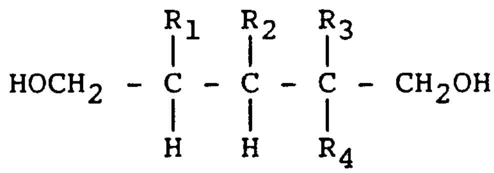

wherein $R_1$ is hydrogen or linear or branched alkyl having from 1 to 3 carbon atoms, and $R_2$, $R_3$ and $R_4$ are the same or different and are hydrogen or linear or branched alkyl having from 1 to 4 carbon atoms, said liquid hydrocarbon diol existing as a liquid at a temperature of 35°C or less; provided (a) at least 2 of $R_1$, $R_2$, $R_3$ and $R_4$ are other than hydrogen; (b) the total number of carbon atoms in $R_1$, $R_2$, $R_3$ and $R_4$ together is 5 or more except as follows: (i) when $R_2$ is ethyl and one of $R_1$, $R_3$ and $R_4$ is methyl, then the remaining 2 of $R_1$, $R_3$ and $R_4$ can be hydrogen; (ii) when $R_3$ and $R_4$ are independently methyl and propyl, then $R_1$ and $R_2$ can be hydrogen; and (iii) when $R_1$ is methyl and $R_2$ is ethyl and one of $R_3$ and $R_4$ is methyl, then the remaining $R_3$ or $R_4$ can be hydrogen; (c) when the total number of carbon atoms in $R_1$ and $R_2$ is 4 or more, then the total number of carbon atoms in $R_2$ is 3 or less except when $R_1$ is propyl and $R_3$ and $R_4$ are hydrogen, then $R_2$ can be butyl; and (d) when $R_1$ is ethyl and $R_2$ is propyl, then $R_3$ is other than ethyl; and to derivatives of said liquid hydrocarbon diols.

As further indicated above, this invention relates to a process comprising:

(a) contacting a substituted vinyl ether with a substituted or unsubstituted acrolein to form a substituted 3,4-dihydropyran;

(b) contacting the substituted 3,4-dihydropyran with an acid catalyst to form a substituted dialdehyde; and

(c) hydrogenating the substituted dialdehyde in the presence of a catalyst to form a liquid hydrocarbon diol represented by the formula:

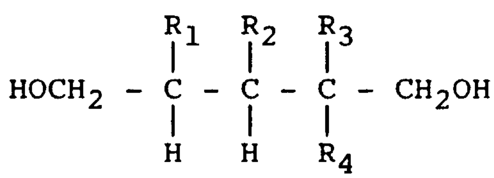

wherein $R_1$ is hydrogen or linear or branched alkyl having from 1 to 3 carbon atoms, and $R_2$, $R_3$ and $R_4$ are the same or different and are hydrogen or linear or branched alkyl having from 1 to 4 carbon atoms, said liquid hydrocarbon diol existing as a liquid at a temperature of 35°C or less; provided (a) at least 2 of $R_1$, $R_2$, $R_3$ and $R_4$ are other than hydrogen; (b) the total number of carbon atoms in $R_1$, $R_2$, $R_3$ and $R_4$ together is 5 more except as follows: (i) when $R_2$ is ethyl and one of $R_1$, $R_3$ and $R_4$ is methyl, then the remaining 2 of $R_1$, $R_3$ and $R_4$ can be hydrogen; (ii) when $R_3$ and $R_4$ are independently methyl and propyl, then $R_1$ and $R_2$ can be hydrogen; and (iii) when $R_1$ is methyl and $R_2$ is ethyl and one of $R_3$ and $R_4$ is methyl, then the remaining $R_3$ or $R_4$ can be hydrogen; (c) when the total number of carbon atoms in $R_1$ and $R_2$ is 4 or more, then the total number of carbon atoms in $R_2$ is 3 or less except when $R_1$ is propyl and $R_3$ and $R_4$ are hydrogen, then $R_2$ can be butyl; and (d) when $R_1$ is ethyl and $R_2$ is propyl, then $R_3$ is other than ethyl.

More particularly, it has been found that low density, liquid 1,5-pentanediols represented by Formula I above can be prepared by reacting acrolein or substituted acrolein with a substituted vinyl ether as follows:

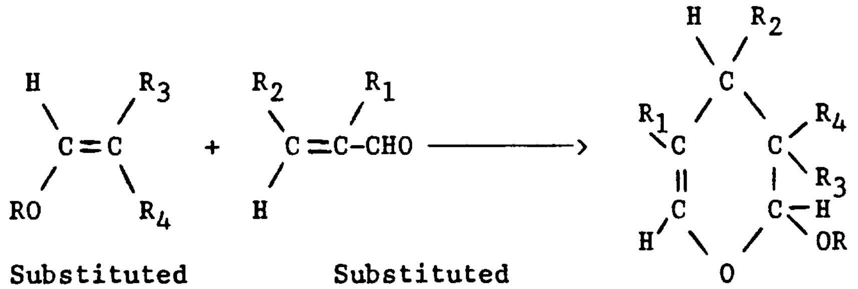

Substituted Vinyl Ether

Substituted Acrolein

Substituted 3,4-Dihydropyran

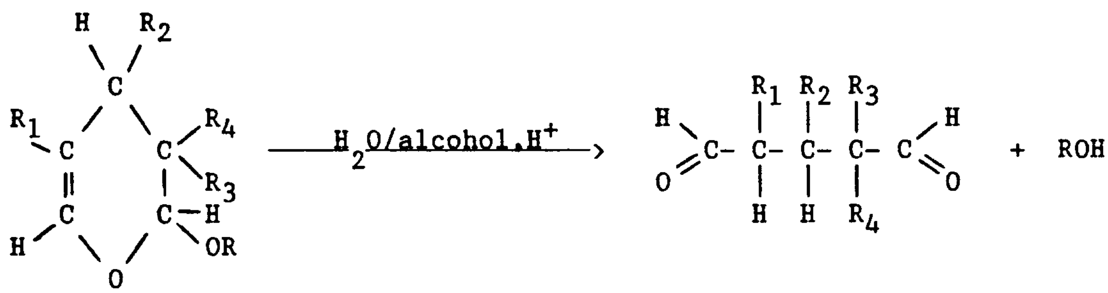


Substituted 3,4-Dihydropyran

Substituted Dialdehyde

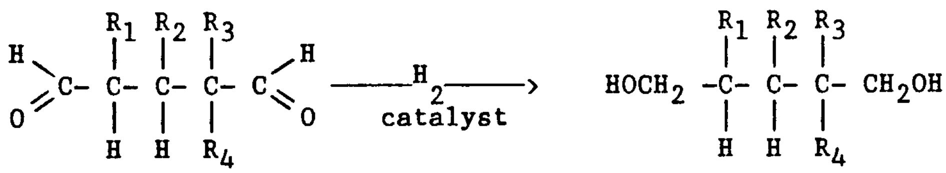


Substituted Dialdehyde

Substituted 1,5-Pentanediol

wherein $R_1$, $R_2$, $R_3$ and $R_4$ are as defined above and R is a substituted or unsubstituted hydrocarbon residue, preferably a linear or branched alkyl having from 1 to about 8 carbon atoms.

Illustrative of suitable substituted vinyl ethers useful in the processes of this invention include, among others, alkyl vinyl ethers such as methyl vinyl ether; methyl (2-methyl vinyl) ether which has the structure:

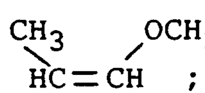

;

methyl (2-ethyl vinyl) ether, which has the structure:

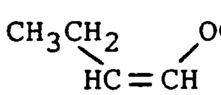

;

methyl (2,2-dimethyl vinyl) ether; methyl (2-methyl, 2-propyl vinyl) ether; methyl (2-butyl, 2-methyl vinyl) ether; ethyl vinyl ether; ethyl (2-methyl vinyl); ethyl (2-ethyl vinyl) ether; ethyl (2,2-dimethyl vinyl)

ether; ethyl (2-methyl, 2-propyl vinyl) ether; ethyl (2-butyl, 2-methyl vinyl) ether; n-propyl and i-propyl vinyl ethers; butyl vinyl ethers such as n-butyl vinyl ether, s-butyl vinyl ether, i-butyl vinyl ether, and t-butyl vinyl ether; amyl vinyl ethers, and the like; divinyl ethers such as triethylene glycol divinyl ether, 1,4-cyclohexane dimethanol divinyl ether, trivinyl ethers, and the like. It is preferred that alkyl vinyl ethers with up to 3 carbon alkyl groups and one- to three-carbon alkyl (alkyl vinyl) ethers with alkyl vinyl groups of up to 8 carbon atoms are used.

Illustrative of suitable acroleins useful in the processes of this invention include, among others, acrolein; 2-ethyl-2-butenal; 2-methyl-2-butenal; 2-(n-propyl)-2-butenal; 2-(i-propyl)-2-butenal; 2-methyl-2-pentenal, which has the structure:

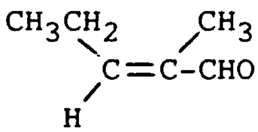

;

2-ethyl-2-pentenal; 2-(n-propyl)-2-pentenal; 2-(i-propyl)-2-pentenal; 2-(n-butyl)-pentenal; 2-(i-butyl)-pentenal; 2-(s-butyl)-pentenal; 2-(t-butyl)-pentenal; 2-amyl pentenals; 2-ethyl-2-hexenal, which has the structure:

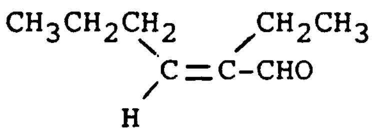

;

2-methyl-2-hexenals; 2-(n-propyl)-2-hexenals; 2-(i-propyl)-2-hexenals; 2-(n-butyl)-2-hexenals; 2-(i-butyl)-2-hexenals; 2-(s-butyl)-2-hexenals; 2-(t-butyl)-2-hexenals; 2-amyl hexenals; and the like.

Illustrative of suitable substituted 3,4-dihydropyrans prepared in reaction step (a) above include, for example, 2-alkoxy-5-ethyl-4-propyl-3,4-dihydro-1,2-pyran, 2-alkoxy-4-ethyl-5-methyl-3,4-dihydro-1,2-pyran, 2-alkoxy-4-ethyl-3,5-dimethyl-3,4-dihydro-1,2-pyran, 2-alkoxy-5-ethyl-3-methyl-4-propyl-3,4-dihydro-1,2-pyran, 2-alkoxy-3,4-diethyl-5-methyl-3,4-dihydro-1,2-pyran, 2-alkoxy-4-ethyl-3,3',5-trimethyl-3,4-dihydro-1,2-pyran, 2-alkoxy-3,3'-dimethyl-4-propyl-3,4-dihydro-1,2-pyran, 2-alkoxy-3-methyl-3'-propyl-3,4-dihydro-1,2-pyran, 2-alkoxy-4-ethyl-5-methyl-3-methyl-3'-propyl-3,4-dihydro-1,2-pyran, 2-alkoxy-5-ethyl-3-methyl-3',4-dipropyl-3,4-dihydro-1,2-pyran, 2-alkoxy-3-butyl-3'-ethyl-3,4-dihydro-1,2-pyran, 2-alkoxy-3-butyl-3',4-diethyl-5-methyl-3,4-dihydro-1,2-pyran, 2-alkoxy-3-butyl-3',5-diethyl-4-propyl-3,4-dihydro-1,2-pyran, and the like. For purposes of these illustrative substituted 3,4-dihydropyrans, alkoxy refers to methoxy, ethoxy, n-propoxy, isopropoxy, n-butoxy, i-butoxy, s-butoxy, t-butoxy, and the like.

The step (a) reaction can be conducted at a temperature of from about 160°C to 280°C for a period of about 1 hour to about 7 days with the longer time being used at the lower temperature, preferably from about 180°C to about 270°C for about 1 hour to about 5 days, and more preferably at about 200°C to 260°C for about 1 hour to about 48 hours. During the reaction, from less than 0.01 percent by weight to about 5 percent by weight of the total weight of the starting materials, preferably from about 0.01 percent by weight to about 2 percent by weight, of a free radical inhibitor can be added to the reaction mass. Illustrative of such free radical inhibitors are 2,6-ditertiarybutyl-4-methyl phenol, hydroquinone, hydroquinone monomethyl ether, and the like. A particularly useful inhibitor is hydroquinone.

The step (a) reaction can be conducted over a wide range of pressures ranging from atmospheric pressure to superatmospheric pressures, e.g., from about 1 atmosphere to about 100 atmospheres or greater. It is preferable to conduct the step (a) reaction at pressures of from about atmospheric to about 75 atmospheres. The step (a) reaction is preferably effected in the liquid or vapor states or mixtures thereof.

The molar ratio of substituted vinyl ether to acrolein compound in the step (a) reaction is not narrowly critical and can range from about 0.05:1 or less to about 50:1 or greater, preferably from about 0.1:1 to about 10:1.

Illustrative of acid catalysts suitable for ring opening of the substituted 3,4-dihydropyran intermediates to form substituted dialdehydes in accordance with reaction step (b) above are mineral acids, including sulfuric acid, hydrochloric acid, phosphoric acid, triflic acid and its salts, sulfonic acids; organic acids including acetic acid, chloroacetic acid, oxalic acid; crosslinked acidic resins such as the various ion exchange resins including Amberlite® CG-400, Amberlite® IR-118; Amberlite® IR120(plus), Dowex® MSC-1, Dowex® M-31, Dowex® M32, Dowex® 50X2-100, Dowex® 50X2-200, Dowex® 50X2-400, Dowex® 50X4-400, Dowex® 50X8-100, Dowex® 50X8-200, Dowex® 50X8-400, Nafion® 117, Nafion® 417, Nafion® NR50, Nafion® perfluorinated powder, similar crosslinked acidic resins; perfluorinated polymers which contain sulfonic acid groups such as XUS-40036.02 (Dow Chemical Company); and the like. The illustrative ion exchange resins above, as well as others, are available from Aldrich Chemical Company, Inc.

The acid catalysts employed in the ring-opening of the substituted 3,4-dihydropyran intermediates are preferably used in conjunction with water; alcohols such as methanol, ethanol, isopropanol, n-propanol, n-butanol, sec-butanol, isobutanol, and tert-butanol, amyl alcohols as well as higher alcohols; glycol ethers such as ethoxy ethanol, methoxy ethanol, 1-methoxypropane, methoxyethoxy ethanol; glyme; and the like, as well as mixtures of water and other solvents. The amount of acid catalyst used in reaction step (b) is dependent on the particular catalyst employed and can range from about 0.01 weight percent or less to about 10 weight percent or greater of the total weight of the starting materials.

The acid dialdehyde reaction mass can be washed with water and/or aqueous solution of neutralizing agents. Illustrative of such neutralizing agents are sodium acetate, potassium acetate, sodium bicarbonate, sodium carbonate, potassium bicarbonate, potassium carbonate, and the like.

Illustrative of suitable substituted dialdehydes prepared in reaction step (b) above include, for example, 3-ethyl-2-methyl-1,5-pentanedial, 2-ethyl-3-propyl-1,5-pentanedial, 3-ethyl-2,4-dimethyl-1,5-pentanedial, 2-ethyl-4-methyl-3-propyl-1,5-pentanedial, 3,4-diethyl-2-methyl-1,5-pentanedial, 3-ethyl-2,4,4'-trimethyl-1,5-pentanedial, 2-ethyl-4,4'-dimethyl-3-propyl-1,5-pentanedial, 2-methyl-2'-propyl-1,5-pentanedial, 3-ethyl-2,4-dimethyl-4'-propyl-1,5-pentanedial, 2-ethyl-4-methyl-3,4'-dipropyl-1,5-pentanedial, 2-butyl-2'-ethyl-1,5-pentanedial, 4-butyl-3,4-diethyl-2-methyl-1,5-pentanedial, 4-butyl-2,4'-diethyl-3-propyl-1,5-pentanedial, and the like.

The step (b) reaction can be conducted over a wide range of pressures ranging from atmospheric or subatmospheric pressures to superatmospheric pressures, e.g., from about 1 atmosphere or less to about 25 atmospheres or greater. It is preferable to conduct the step (b) reaction at pressures of from about 1 atmosphere to about 10 atmospheres. The step (b) reaction is preferably effected in the liquid or vapor states or mixtures thereof.

The temperature of the step (b) reaction may be as low as about ambient temperature to about 300°C. Preferably, the reaction temperature ranges from about 50°C to about 200°C, and more preferably from about 60°C to about 120°C.

Illustrative of catalysts useful in the hydrogenation step (c) involving reduction of substituted dialdehydes include, for example, Raney-type compounds such as Raney nickel and modified Raney nickels; molybdenum-promoted nickel, chromium-promoted nickel, cobalt-promoted nickel; platinum; palladium; iron; cobalt molybdate on alumina; copper chromite; barium promoted copper chromite; tin-copper couple; zinc-copper couple; aluminum-cobalt; aluminum-copper; and aluminum-nickel; platinum; nickel; and the like. The amount of hydrogenation catalyst used in step (c) is dependent on the particular catalyst employed and can range from about 0.01 weight percent or less to about 10 weight percent or greater of the total weight of the starting materials.

The particular reaction conditions for the step (c) hydrogenation reaction are not narrowly critical, and can be any effective hydrogenation procedures sufficient to produce the substituted 1,5-pentanediols of this invention. The step (c) reaction can be carried out at temperatures of from about ambient temperature to about 250°C, preferably from about 70°C to about 200°C, and more preferably from 90°C to 150°C. The step (c) reaction preferably may be conducted at pressures of from about 5 atmospheres to about 100 atmospheres, and more preferably from about 10 atmospheres to about 75 atmospheres.

The products produced by the processes of this invention include substituted 1,5-pentanediols, particularly those substituted 1,5-pentanediols encompassed within Formula I, which are useful in a number of ways including imparting excellent physical characteristics, such as water resistance, chemical resistance and the like, to coatings, inks, adhesives, and sealants prepared from the substituted 1,5-pentanediols or derivatives thereof.

Illustrative of suitable substituted 1,5-pentanediols prepared by the processes of this invention include, for example, 3-ethyl-2-methyl-1,5-pentanediol, 2-ethyl-3-propyl-1,5-pentanediol, 2,4-dimethyl-3-ethyl-1,5-pentanediol, 2-ethyl-4-methyl-3-propyl-1,5-pentanediol, 2,3-diethyl-4-methyl-1,5-pentanediol, 3-ethyl-2,2,4-trimethyl-1,5-pentanediol, 2,2-dimethyl-4-ethyl-3-propyl-1,5-pentanediol, 2-methyl-2-propyl-1,5-pentanediol, 2,4-dimethyl-3-ethyl-2-propyl-1,5-pentanediol, 2,3-dipropyl-4-ethyl-2-methyl-1,5-pentanediol, 2-butyl-2-ethyl-1,5-pentanediol, 2-butyl-2,3-diethyl-4-methyl-1,5-pentanediol, 2-butyl-2,4-diethyl-3-propyl-1,5-pentanediol, 3-butyl-2-propyl-1,5-pentanediol and the like.

The substituted 1,5-pentanediol products produced by the processes of this invention can be separated by distillation. For example, a crude reaction product can be subjected to a distillation-separation at atmospheric or reduced pressure through a packed distillation column. Reactive distillation may be useful in conducting certain reaction steps of this invention.

The processes of this invention may be carried out using, for example, a fixed bed reactor, a fluid bed reactor, or a slurry reactor. The optimum size and shape of the catalysts will depend on the type of reactor used. In general, for fluid bed reactors, a small, spherical catalyst particle is preferred for easy fluidization. With fixed bed reactors, larger catalyst particles are preferred so the back pressure within the reactor is kept reasonably low.

The processes of this invention can be conducted in a batch or continuous fashion, with recycle of unconsumed starting materials if required. The reaction can be conducted in a single reaction zone or in a plurality of reaction zones, in series or in parallel or it may be conducted batchwise or continuously in an elongated tubular zone or series of such zones. The materials of construction employed should be inert to the starting materials during the reaction and the fabrication of the equipment should be able to withstand the reaction temperatures and pressures. Means to introduce and/or adjust the quantity of starting materials or ingredients introduced batchwise or continuously into the reaction zone during the course of the reaction can be conveniently utilized in the processes especially to maintain the desired molar ratio of the starting materials. The reaction steps may be effected by the incremental addition of one of the starting materials to the other. Also, the reaction steps can be combined by the joint addition of the starting materials. When complete conversion is not desired or not obtainable, the starting materials can be separated from the product, for example by distillation, and the starting materials then recycled back into the reaction zone.

The processes are conducted for a period of time sufficient to produce the substituted 1,5-pentanediol products. The exact reaction time employed is dependent, in part, upon factors such as temperature, nature and proportion of starting materials, and the like. The reaction time will normally be within the range of from about one-half to about 100 hours or more, and preferably from less than about one to about ten hours.

The process may be conducted in either glass lined, stainless steel or similar type reaction equipment. The reaction zone may be fitted with one or more internal and/or external heat exchanger(s) in order to control undue temperature fluctuations, or to prevent any possible "runaway" reaction temperatures.

The substituted 1,5-pentanediols produced by the processes of this invention, including the substituted 3,4-dihydropyran and substituted dialdehyde intermediates, can undergo further reaction(s) to afford desired derivatives thereof. Such permissible derivatization reactions can be carried out in accordance with conventional procedures known in the art. Illustrative derivatization reactions include, for example, esterification, etherification, alkoxylation, amination, alkylation, hydrogenation, dehydrogenation, reduction, acylation, condensation, carboxylation, oxidation, silylation and the like, including permissible combinations thereof. This invention is not intended to be limited in any manner by the permissible derivatization reactions or permissible derivatives of liquid hydrocarbon diols.

More particularly, the substituted 1,5-pentanediols of this invention can undergo any of the known reactions of hydroxyl groups illustrative of which are reactions with acyl halides to form esters; with ammonia, a nitrile, or hydrogen cyanide to form amines; with alkyl acid sulfates to form disulfates; with carboxylic acids and acid anhydrides to form esters and polyesters; with alkali metals to form salts; with ketenes to form esters; with acid anhydrides to form carboxylic acids; with oxygen to form aldehydes and carboxylic acids; ring-opening reactions with lactones, tetrahydrofuran, and alkylene oxides such as ethylene oxide, propylene oxide, epichlorohydrin; dehydrogenation to form aldehydes, isocyanates to form urethanes, and the like.

Products containing free carboxylic acid groups can be made by conventional methods by reacting the substituted 1,5-pentanediols of Formula I with acid anhydrides which may be illustrated by the following reaction in which one equivalent of acid anhydride is used for each equivalent of hydroxyl:

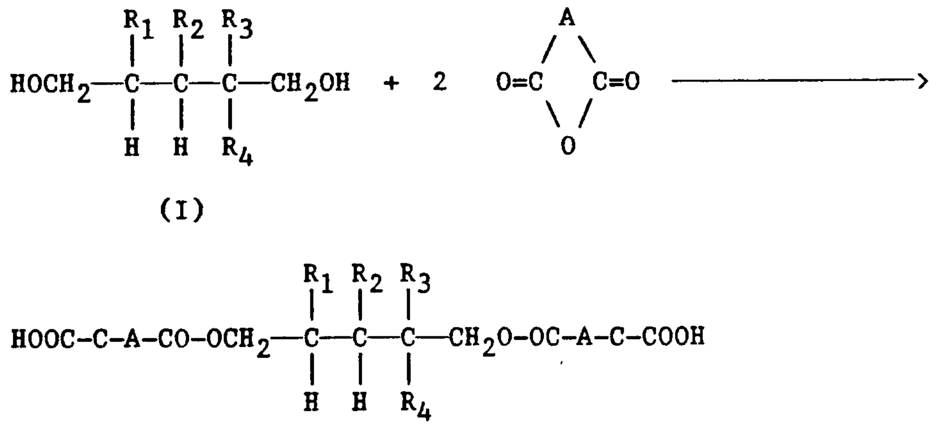

(I)

(II)

wherein $R_1$, $R_2$, $R_3$ and $R_4$ are as defined above and A is the same or different and is a substituted or unsubstituted hydrocarbon residue, i.e., a residue of an acid anhydride when the anhydride group is removed. Illustrative of A substituents include $(CH_2)_4$ when the anhydride of adipic acid is used, $C_6H_4$ when phthalic anhydride is used, $C_6H_8$ when hexahydrophthalic anhydride is used, and the like.

The product of Formula II can be prepared by heating the product of Formula I and an acid anhydride at a temperature of about 60 ° C to about 200 ° C, preferably from about 80 ° C to about 160 ° C, and most preferably from about 100 ° C to about 140 ° C for a period of time ranging from about 30 minutes to about 8 hours or more, preferably from about 1 hour to about 4 hours, or until substantially all of the acid anhydride has reacted with the hydroxyl groups. Usually about 0.10 to about 1.0 equivalent of anhydride are used per equivalent of hydroxyl, preferably about 0.25 to about 1.0 equivalent of anhydride per equivalent of hydroxyl, and most preferably from about 0.85 to about 1.0 equivalent of anhydride per equivalent of hydroxyl. If desired, the reaction may be carried out in the presence of a solvent that is inert to reaction with hydroxyl groups or anhydride groups such as benzene, toluene, methyl ethyl ketone, methyl i-butyl ketone, methyl amyl ketone, ethoxyethyl acetate, ethoxyethyl butyrate, and the like.

Illustrative of the anhydrides that can be used in preparation of the Formula II free carboxylic acid products include, for example, tetrahydrophthalic anhydride, phthalic anhydride, isophthalic anhydride, benzophenone dicarboxylic acid anhydride, succinic anhydride, glutaric anhydride, napthoic anhydride, chlorendic anhydride, maleic anhydride, itaconic anhydride, or another intramolecular anhydride including those having substituents thereon such as alkyl or alkoxy groups, nitro, halogen, aryl, carboxyl, or any other group that will not unduly interfere with the reaction, and the like as well as mixtures of anhydrides.

If desired, a catalyst may be used in preparation of the Formula II free carboxylic acid products. Illustrative of the catalysts that can be used to prepare the Formula II free carboxylic acid products of this invention are dibutyltin dioxide, antimony oxide, tin oxide, titanium alkoxides, alkali metal salts or metallic salts of manganese, cadmium, magnesium, zinc, tin, and the like.

The substituted 1,5-dicarboxylic acids of Formula II and also Formula IV below can be used in combination with polyols, including Formula I diols, having two or more hydroxyl groups to produce polyesters that can be used in coatings, adhesives, inks, fibers, fabrics, shaped articles, and the like.

Ester products can be prepared by conventional methods by reacting Formula I compounds with an anhydride such as acetic anhydride which would form the dimethyl ester when one equivalent of acetic anhydride is reacted with each equivalent of Formula I diol:

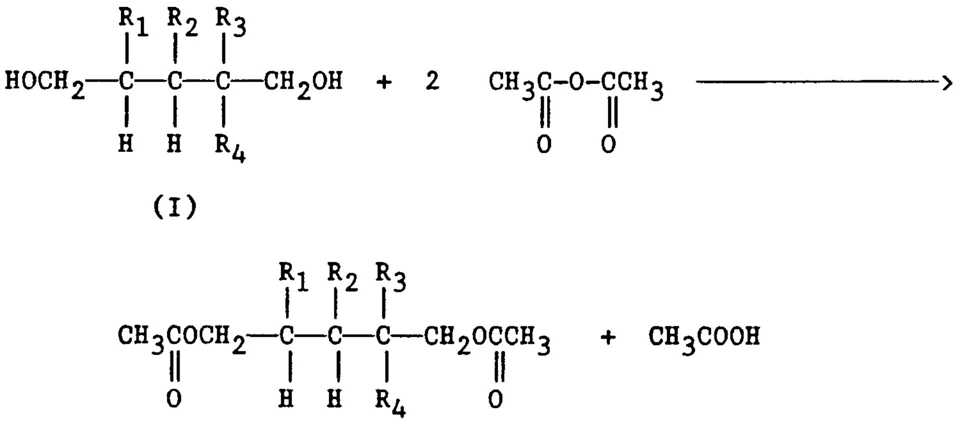

(I)

(III)

wherein $R_1$, $R_2$, $R_3$ and $R_4$ are as defined above. It is understood by those skilled in the art that less than a 1:1 equivalency of anhydride per hydroxyl group will result in a partially esterified product that will contain both ester and hydroxyl groups. Esterified and partially esterified products such as these are useful as inert solvents or intermediates for further reaction through free hydroxyl groups or through transesterification with suitable compounds including themselves.

Silicone-containing compounds can be prepared by conventional methods by either end capping, coupling, or other reaction when Formula I diols or mixtures of Formula I diols and optionally other polyols are reacted with silanes. Illustrative of the silane-containing compounds are the following:

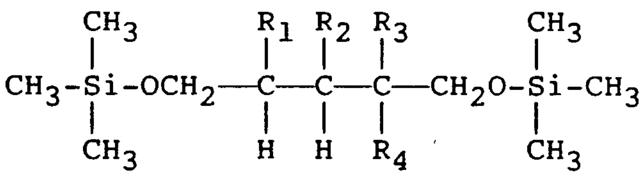

when 2 moles of trimethylchlorosilane and 1 mole of Formula I diol are reacted,

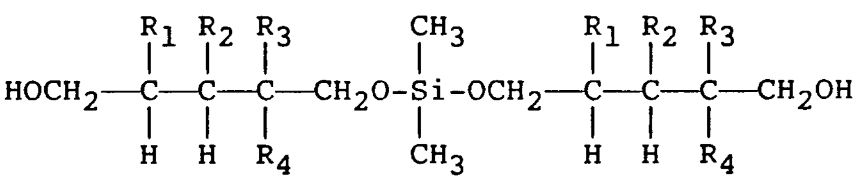

when one mole of dimethyldichlorosilane and two moles of Formula I diol are reacted,

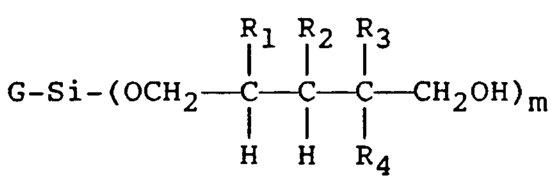

wherein $R_1$, $R_2$, $R_3$ and $R_4$ are as defined above, m is 3 or 4 and G is methyl when m is 3 and nonexistent when m is 4 when 3 moles (m = 3) or 4 moles (m = 4) of Formula I diol are reacted with methyltrichlorosilane or tetrachlorosilane respectively, and the like. It is understood by those skilled in the art that when polyfunctional compounds are combined, a variety of products, including chain extended products, can be obtained. Illustrative of the silanes include, for example, chloroalkylchloro and arylchlorosilanes, diphenylethylchlorosilane, trimethylchlorosilane, dimethyldichloromethylsilane, triphenylchlorosilane, methyldichlorsilane, dimethylethylchlorosilane, dichlorosilane; alkoxysilanes such as methoxysilane, dimethoxysilane, diethyoxysilane, triethoxysilane, dimethylmethoxychlorsilane, dimethylmethoxysilane, tris(methoxy)-3-chloropropylsilane, and the like.

The silicone-containing compounds can be used as solvents, surfactants, reactive diluents or other components, as hydraulic fluids, as intermediates for the formation of other compounds, as components in polyesters that have utility in coatings, inks, adhesives, fibers, and molded articles, as well as in other end uses.

Illustrative polyols that can be used in combination with the Formula I diols to prepare derivatives thereof include, for example, diethylene glycol, 1,4-butanediol, 1,6-hexanediol, 1,4-dihydroxyquinone, 2,2-dimethyl-1,3-propanediol, hydroxyl-terminated polyesters, ethylene oxide/propylene oxide copolymer polyols, poly(ethylene oxide) polyols, poly(propylene oxide) polyols, poly(alkylene oxide) polyols, poly(tetramethylene oxide) polyols, polyether polyols, polycarbonate polyols, polylactone polyols, and the like, including mixtures thereof.

Polylactone polyols can be prepared by conventional methods by reacting from 1 to 50 moles, preferably from 1 to 25, and most preferably from 1 to 10 moles of a lactone with one mole of one of the substituted 1,5-pentanediols of this invention. Illustrative of the lactones that can be used include, for example, $\epsilon$-caprolactone, $\epsilon$-methyl-$\epsilon$-caprolactone, $\gamma$-methyl-$\epsilon$-caprolactone, $\gamma$-methyl-$\epsilon$-methyl-$\epsilon$-caprolactone, $\delta$-valerolactone and alkyl or aryl substituted $\delta$-valerolactones, $\delta$-enantolactone and alkyl or aryl substituted $\delta$-enantolactone, $\beta$-propriolactone, and the like. The polylactone polyols are prepared by heating the lactone and substituted 1,5-pentanediol at a temperature of about 140°C to about 210°C, preferably from about 150°C to about 180°C for a period of time of about 6 hours to about 36 hours, preferably from 8 hours to about 24 hours. It is preferred to use from about 10 to 5000 or more parts per million, preferably from about 20 to about 1500 parts per million, of a catalyst to promote the ring-opening polymerization. Illustrative of such catalysts are the organometallics such as stannous octanoate, dibutyltin dilaurate, zinc octanoate, and the like. Such polylactone polyols have many uses, illustrative of which are intermediates for the preparation of polyurethane elastomers and foams, aminoplast-cured coatings, radiation-cured coatings and inks, and the like. Use of the substituted 1,5-pentanediols of this invention in preparation of the polyols will result in improved hydrolytic stability for materials prepared from the polylactone polyols and is expected to produce polyols that remain liquid below about 30°C at molecular weights of about 1000 or more.

In an embodiment of this invention, certain substituted 3,4-dihydropyran intermediates can be isolated and used in a variety of ways including as reactive diluents for cycloaliphatic epoxide systems that are curable by either thermal means when cation-generating catalysts/initiators such as boron trifluoride, boron trifluoride etherate, triflic acid or its salts such as diethyl ammonium triflate, ammonium triflate, and the like are used, or by photochemical means when photolyzable Bronsted or Lewis acid-generating photoinitiators such as the onium salts which can be illustrated by the arylsulfonium hexafluorophosphates, the arylsulfonium hexafluoroarsenates, the arylsulphonium hexafluoroantimonates, diazonium hexafluorophosphate, iodonium hexafluorophosphate, iodonium hexafluoroantimonates, and the like are used.

In another embodiment of this invention, the substituted dialdehyde intermediates can be isolated for use as pharmaceutical intermediates, as compounds that can be oxidized to the corresponding dicarboxylic acids, and modified to amines. Illustrative of such use of the substituted dialdehydes is provided by the following reaction scheme:

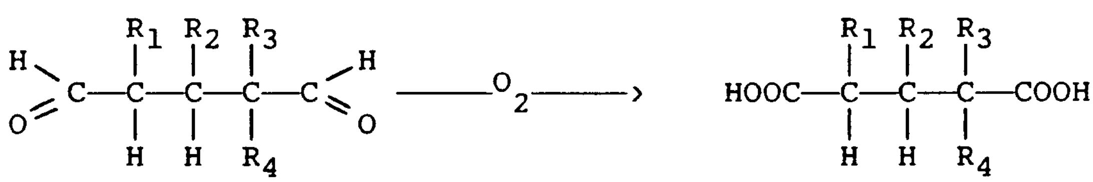

catalyst

(IV)

wherein $R_1$, $R_2$, $R_3$ and $R_4$ are as defined above and substituted 1,5-dicarboxylic acids of Formula IV are formed. Such compounds are useful in a variety of ways including reaction of one mole of the substituted 1,5-dicarboxylic acid with two moles of tetrahydrobenzyl alcohol and subsequent epoxidation with a peracid such as peracetic acid or perpropionic acid to the corresponding cycloaliphatic epoxides as

illustrated by the following reaction scheme:

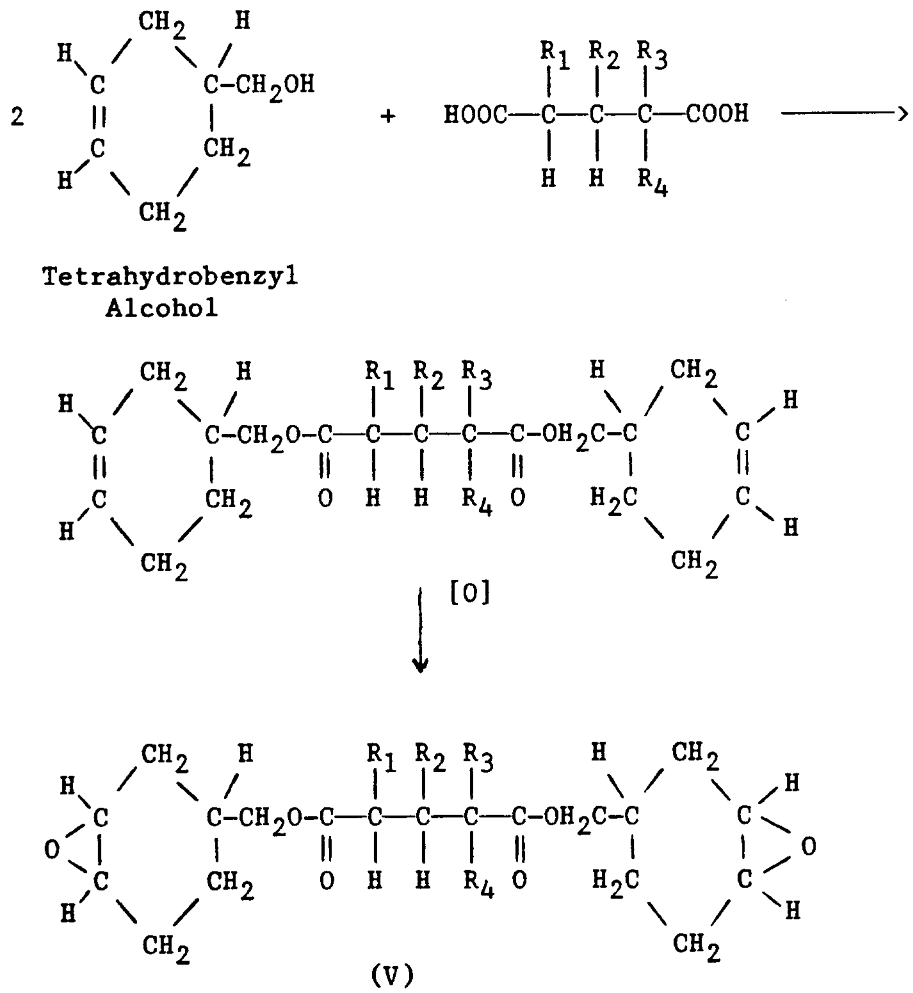


Tetrahydrobenzyl Alcohol

(V)

wherein $R_1$, $R_2$, $R_3$ and $R_4$ are as defined above.

Formula V cycloaliphatic epoxides can be crosslinked into useful coatings, inks, and sealants if formulated with Formula II and/or Formula IV carboxylic acid containing products in ratios of from about 1.5 equivalents of epoxide or more per equivalent of carboxylic acid. Such formulations can be cured at temperatures of about 35 ° C to 200 ° C for periods of times of from about 5 minutes to 24 hours or longer. It is preferable to use an alkyl metal catalyst; illustrative of which are stannous octanoate, dibutyl tin dilaurate, and the like; or Bronsted acids such as triflic acid salts; illustrative of which are diethylammonium triflate, ammonium triflate, diisopropylammonium triflate, and the like, to minimize curing time.

As used herein, the term "polyol" is contemplated to include all permissible hydrocarbon compounds having 2 or more hydroxyl groups, e.g., diols, triols and the like.

For purposes of this invention, the term "hydrocarbon" is contemplated to include all permissible compounds having at least one hydrogen and one carbon atom. In a broad aspect, the permissible hydrocarbons include acyclic and cyclic, branched and unbranched, carbocyclic and heterocyclic, aromatic and nonaromatic organic compounds which can be substituted or unsubstituted.

As used herein, the term "substituted" is contemplated to include all permissible substituents of organic compounds unless otherwise indicated. In a broad aspect, the permissible substituents include acyclic and cyclic, branched and unbranched, carbocyclic and heterocyclic, aromatic and nonaromatic substituents of organic compounds. Illustrative substituents include, for example, alkyl, alkyloxy, aryl, aryloxy, hydroxy, hydroxyalkyl, amino, aminoalkyl, halogen and the like in which the number of carbons can range from 1 to about 20 or more, preferably from 1 to about 12. The permissible substituents can be one or more and the same or different for appropriate organic compounds. This invention is not intended to be limited in any manner by the permissible substituents of organic compounds.

Certain of the following examples are provided to further illustrate this invention.

Glossary of Terms

Gloss Retention – This gloss retention is a measure of water resistance. After measuring the 20° spectral gloss, the coated panels were suspended in a 50°C water bath for 17 hours. They were removed from the bath, dried, and the 20° spectral gloss was remeasured and the percent gloss retention was calculated.

Spot Tests (5 hour) – Spot tests were performed by using 5 percent hydrochloric acid, 20 percent sodium hydroxide, bleach, mustard, lipstick, iodine, and ink by placing an approximately one-inch diameter drop on the coating surface and allowing the "spotted" sample to remain uncovered under ambient condition for 5 hours. The test solution was then rinsed away using deionized water or by wiping in the case of solids, and the coating was examined for blushing, blistering, or discoloration.

Double Rubs – Solvent resistance was measured as the number of solvent (methyl ethyl ketone double rubs or acetone double rubs) that were required to cut through the coating. If 100 rubs or more did not cut through the coating, the coating was recorded as >100. To perform the test, the solvent-soaked cloth was rubbed back and forth with hand pressure. A rub back and forth was designated as one "double rub."

Crosshatch Adhesion – Procedure conducted in accordance with ASTM D 3359-87.

Conical Mandril Bend – Procedure conducted in accordance with ASTM D 522-85.

Pencil Hardness – Procedure conducted in accordance with ASTM D 3363-74.

Electrical Insulation Resistance – This electrical property is a measure of the electrical resistance property or resistance offered by the material to passage of an electrical current. The measurements were made in accordance with method 302, test condition B, of Military Standard 202. Insulating materials are very poor conductors of electricity and offer high resistance and thus the higher the value, the better the insulating property of a material. When temperature increases, insulating resistance decreases. When moisture content of a coating as might be encountered under high humidity conditions increases, insulation resistance decreases. Coatings with a high insulation resistance and the ability to maintain such resistance under high humidity conditions are useful protectors of electrical circuitry.

Distinctness of Reflected Image – This property was measured with a Model #1792 Portable Distinctness of Reflected Image Meter using the manufacturer's method of operation. The meter is available from ATI Systems Inc., Madison Heights, MI.

Acrylic I – A thermoplastic acrylic polymer for formulation of coatings and commercially available from Rohm and Haas Company as AT-954.

Catalyst I – A 40 percent solution of p-toluenesulfonic acid in methanol.

Catalyst II – A solution of diethylammonium triflate commercially available from 3M Company as FC-520.

Epoxide I – A cycloaliphatic epoxide commercially available from Union Carbide Chemicals and Plastics Company Inc. as CYRACURE® UVR-6110, 3,4-epoxycyclohexylmethyl 3,4-epoxycyclohexane-carboxylate.

Melamine I – A polymethoxymethyl melamine commercially available from Monsanto Company as CYMEL® 303.

Surfactant I – A 25 percent solution of a methoxy-terminated poly(ethylene oxide) modified polydimethylsiloxane in methyl amyl ketone commercially available from Union Carbide Chemicals and Plastics Company Inc. as SILWET® L-77.

Surfactant II – A 25 percent solution of a methoxy-terminated ethylene oxide/propylene oxide copolymer modified polydimethylsiloxane in methyl amyl ketone commercially available from Union Carbide Chemicals and Plastics Company Inc. as SILWET® L-7001.

Surfactant III – A hydroxy-terminated poly(ethylene oxide) modified polydimethylsiloxane commercially available from Union Carbide Chemicals and Plastics Company Inc. as SILWET® L-7604.

Photoinitiator I – A cationic photoinitiator commercially available from Union Carbide Chemicals and Plastics Company Inc. as CYRACURE® UVI-6974.

Vinyl Ester I – A vinyl ester monomer commercially available from Union Carbide Chemicals and Plastics Company Inc. as VYNATE® C10.

Vinyl Ester II – A vinyl ester prepared from Versatic 10 which is a synthetic, saturated monocarboxylic acid with a highly branched structure that contains 10 carbon atoms and is commercially available from Shell Chemical Company as Veova® VV10.

Example 1

Preparation of 2 – ethoxy – 5 – ethyl – 4 – propyl – 3,4 – dihydro – 1,2 – pyran

The following ingredients were charged to a two – gallon, high – pressure, stainless – steel autoclave equipped with an agitator, a pressurizing system, and a temperature sensing device, 1,750 grams (13.9 moles) of 2 – ethyl – 2 – hexenal and 1,316 grams (18.2 moles) of ethyl vinyl ether. The system was purged with nitrogen and pressurized to 200 psig with nitrogen. The reactants were then heated to 260 °C and maintained at this temperature for three hours. After this time, the system was cooled to ambient conditions, and the reaction mass was transferred to a five – liter, round – bottomed flask equipped with a 20 – tray Oldershaw column and a water – cooled automatic reflux head. Unreacted ethyl vinyl ether and 2 – ethyl – 2 – hexenal were recovered by distillation for recycle, and 1,555 grams (7.8 moles) of a product identified by infrared, nuclear magnetic resonance, and mass spectroscopy analyses as 2 – ethoxy – 5 – ethyl – 4 – propyl – 3,4 – dihydro – 1,2 – pyran were then taken overhead (b.p. = 159 °C at 100 torr). The reaction yield and efficiency were 56.1 percent and 94.6 percent, respectively.

Example 2

Preparation of 2 – ethyl – 3 – propyl – 1,5 – pentanediol

To a two – liter, round – bottomed flask equipped with a 20 – tray Oldershaw column and a water – cooled automatic reflux head, 457 grams (2.3 moles) of 2 – ethoxy – 5 – ethyl – 4 – propyl – 3,4 – dihydro – 1,2 – pyran, 393 grams of absolute ethanol, 500 grams of water, and 5.0 grams of concentrated sulfuric acid were added. The mixture was refluxed (85 °C) for 12 hours, and then the ethanol was removed overhead as an azeotrope with water until no more ethanol distilled overhead (12 hours). The mixture remaining in the flask was transferred to a separatory funnel and the water layer was separated from the organic layer and discarded. The organic layer was washed with a saturated solution of sodium bicarbonate, and this was followed by a deionized water wash. The organic layer was then charged to a one – gallon, high – pressure stainless steel autoclave along with 600 grams of absolute ethanol and 40.0 grams of Raney nickel catalyst. The system was purged with nitrogen, pressurized with hydrogen to 1,000 psig, heated to 120 °C for one hour, and then to 150 °C for an additional hour. During the reaction time at the elevated temperatures, the reactor pressure was maintained at 1,000 psig with incremental additions of hydrogen. The system was cooled and the catalyst was separated from the reaction mixture by filtration and the ethanol was removed by evaporation in a Rotavapor. The concentrated mixture was transferred to a 500 milliliter, round – bottomed flask, and using the distillation equipment described above, 308 grams (1.8 moles, 78% yield) of 2 – ethyl – 3 – propyl – 1,5 – pentanediol was taken overhead (b.p. = 159 – 162 °C at 10 torr) as a clear, colorless liquid with a purity of 99% as determined by gas – liquid chromatography. Nuclear magnetic resonance, infrared, and mass spectral analyses were consistent with the structure of the diol.

Example 3

One mole of the 2 – ethyl – 3 – propyl – 1,5 – pentanediol of Example 2, 6 moles of $\epsilon$ – caprolactone, and 60 parts per million stannous octanoate are charged to a stirred reactor equipped with a nitrogen inlet and outlet preferably so placed as to allow the system to be sparged with an inert gas such as dry nitrogen. The stirred ingredients are heated to 100 °C with agitation and held at this temperature for 1 hour to remove moisture, water, or low boiling volatiles from the reaction mixture. Then the temperature is increased to 165 °C and the reaction mass is held at this temperature for 15 hours while blanketing the it with nitrogen. The resulting product is cooled to room temperature and stored for future use.

Example 4

Two moles of phthalic anhydride and one mole of the 2 – ethyl – 3 – propyl – 1,5 – pentanediol are charged to a stirred reactor. The reaction mass is heated to 125 °C while stirring, and held at this temperature for 3 hours. After this time the product is cooled to room temperature and stored for future use.

Example 5

A four-necked, one-liter round bottom flask equipped with a mechanical stirrer, thermometer, nitrogen sparging tube, and a simple distillation head was charged with 76.7 grams (0.44 mole) of 2-ethyl-3-propyl-1,5-pentanediol, 5.4 grams (0.040 mole) of trimethylolpropane, 24.9 grams (0.15 mole) of isophthalic acid, 21.9 grams (0.15 mole) of adipic acid, and 0.26 grams (0.2 weight percent) dibutyltin oxide catalyst. The ingredients were kept under a nitrogen sparge with stirring throughout the course of the reaction. The system was heated gradually heated to 160°C over a one-hour period. The temperature was maintained at 160°C with a Therm-O-Watch controller for one hour, and then the temperature was increased in 10 degree increments every 45 minutes until a reaction temperature of 220°C was attained. This temperature was maintained for 30 minutes after which the system was allowed to cool to room temperature. All water of condensation formed by the reaction was removed with the simple distillation head. The resulting polyester had a Brookfield viscosity of 43,600 centipoise at 23°C, an hydroxyl number of 147, and an acid number of 1.79. Molecular weight determination by gel permeation chromatography using a polystyrene standard resulted in an apparent number average molecular weight of 993, a weight-average molecular weight of 2155, and a polydispersity of 2.17.

Example 6

A polyester was prepared in the same manner as that of Example 5 except the following ingredients were used: 75.2 grams (0.43 mole) of 2-ethyl-3-propyl-1,5-pentanediol, 3.5 grams (0.026 mole) of trimethylolpropane, 16.2 grams (0.10 mole) of isophthalic acid, 14.3 grams (0.10 mole) of adipic acid, and 0.22 grams (0.2 weight percent) dibutyltin oxide catalyst. The resulting polyester had a Brookfield viscosity of 5,440 centipoise at 23°C, an hydroxyl number of 258, and an acid number of 2.23. Molecular weight determination by gel permeation chromatography using a polystyrene standard resulted in an apparent number average molecular weight of 831, a weight-average molecular weight of 1161, and a polydispersity of 1.40.

Control Examples A through D

In the same manner as described in Example 5, control polyesters were prepared from a five-carbon diol, a six-carbon diol, a nine-carbon diol, a four-carbon diol, and the ingredients listed in Table A below. The control polyesters had the properties given in Table A.

Table A

| Ingredients | Control A 5-Carbon Diol grams | moles | Control B 6-Carbon Diol grams | moles | Control C 9-Carbon Diol grams | moles | Control D 4-Carbon Diol grams | moles |
|---|---|---|---|---|---|---|---|---|
| 2,2-dimethyl-1,3-propanediol | 206.8 | 1.98 | — | — | — | — | — | — |
| 2,2,4-trimethyl-1,3-propanediol | — | — | 289.5 | 1.98 | — | — | — | — |
| 2-butyl-2-ethyl-1,3-propanediol | — | — | — | — | 317.3 | 1.98 | — | — |
| 2-methyl-1,3-propanediol | — | — | — | — | — | — | 178.4 | 1.98 |
| Trimethylolpropane | 24.2 | 0.18 | 24.2 | 0.18 | 24.2 | 0.18 | 24.2 | 0.18 |
| Isophthalic acid | 112.0 | 0.67 | 112.0 | 0.67 | 112.0 | 0.67 | 112.0 | 0.67 |
| Adipic acid | 98.4 | 0.67 | 98.4 | 0.67 | 98.4 | 0.67 | 98.4 | 0.67 |
| Dibutyltin oxide | 0.88 | — | 0.88 | — | 0.88 | — | 0.88 | — |
| **Properties** | | | | | | | | |
| Viscosity, cp (23°C) | 314,000 | | 319,000 | | 125,000 | | 39,700 | |
| Molecular Weight (GPC) | | | | | | | | |
| Number average | 910 | | 1124 | | 658 | | 776 | |
| Weight average | 1548 | | 1964 | | 1415 | | 1359 | |
| Equivalent Weight from Hydroxyl Number | 237 | | 420 | | 280 | | 212 | |

Although the polyesters of Control Examples A, B and C had apparent molecular weights as determined by gel permeation chromatography similar to the polyester of Example 5, the viscosities of the Examples 5 and 6 polyesters were markedly lower than that of these control polyesters. The properties of the coatings made from the polyesters of Control Examples A, B, C and D are given in Table C below.

Example 7 and Control Examples E through I

These examples describe preparation of thermally curable coatings prepared from a polyester identified in Table B below and an aminoplast crosslinking agent. The ingredients listed in Table B, except for Catalyst I, were placed in a glass container and thoroughly mixed for each example. When they were well mixed, Catalyst I was stirred into the blend. The blended components were allowed to stand under ambient conditions to allow escape of entrapped air. The liquid coating was then applied with a 10-mil wet-clearance film applicator to Bonderite 40 unpolished, cold-rolled steel panels using the draw-down technique. The coated panels were then baked in an air-circulating oven at 140°C for 30 minutes. The coatings were then tested by the indicated tests in Table C below and the results are given in Table C.

Table B

| Ingredients, grams | Example 7 | Control E | Control F | Control G | Control H | Control I |
|---|---|---|---|---|---|---|
| Example 5 Polyester | 5.3 | --- | --- | --- | --- | --- |
| Control A Polyester | --- | 5.3 | --- | --- | --- | --- |
| Control B Polyester | --- | --- | 5.3 | --- | --- | --- |
| Control C Polyester | --- | --- | --- | 5.3 | --- | --- |
| Control D Polyester | --- | --- | --- | --- | --- | 5.3 |
| Acrylic I | --- | --- | --- | --- | 5.3 | --- |
| Melamine I | 2.3 | 2.3 | 2.3 | 2.3 | 2.3 | 2.3 |
| Methyl amyl ketone | 2.5 | 2.5 | 2.5 | 2.5 | 2.5 | 2.5 |
| Surfactant I | 0.16 | 0.16 | 0.16 | 0.16 | 0.16 | 0.16 |
| Surfactant II | 0.16 | 0.16 | 0.16 | 0.16 | 0.16 | 0.16 |
| Catalyst I | 0.06 | 0.06 | 0.06 | 0.06 | 0.06 | 0.06 |

Table C

| Cured Film Properties | Example 7 | Control E | Control F | Control G | Control H | Control I |
|---|---|---|---|---|---|---|
| Cured Film Thickness, mils | 2.0 | 2.0 | 2.3 | 1.7 | 1.4 | 1.7 |
| Specular Gloss (20°) (ASTM D 523-78) | 91.5 | 91.5 | 95.0 | 90.0 | 89.4 | 90.1 |
| Specular Gloss (60°) (ASTM D 523-78) | 100.4 | 101.6 | 102.0 | 99.4 | 100.2 | 101.8 |
| Pencil Hardness (ASTM D 3363-74) | 4H | F | 2H | F | 4H | F |
| Impact Resistance (ASTM D 2794-84) | | | | | | |
| Forward, in-lbs | 150 | 160 | 150 | 120 | 30 | 120 |
| Reverse, in-lbs | 120 | 50 | 20 | 40 | 5 | 30 |
| Gloss Retention, 20°, % | 100 | 99 | 96.7 | — | 100 | 60 |
| Methyl ethyl ketone double rubs | >100 | >100 | >100 | >100 | >100 | >100 |
| Spot Tests (5 hour)* | | | | | | |
| 5% Hydrochloric Acid | NE | NE | NE | — | — | — |
| 20% Sodium Hydroxide | NE | NE | NE | — | — | — |
| Bleach | NE | NE | NE | — | — | — |
| Ink | NE | NE | NE | — | — | — |
| Lipstick | NE | NE | NE | — | — | — |
| Mustard | SE | NE | NE | — | — | — |
| Iodine | SVE | SE | SE | — | — | — |

*NE = No Effect; SE = Slight Effect; SVE = Severe Effect

The coating of Example 7 has an excellent combination of hardness and both forward and reverse impact resistance along with excellent resistance to acid, base, bleach, ink and lipstick, high gloss and gloss retention when immersed in hot water relative to the coatings prepared from the control polyesters and the control acrylic.

Examples 8 and 9

The following examples describe preparation of thermally curable coatings from the polyester of Example 5 and a cycloaliphatic epoxide in the presence of a triflic acid salt catalyst. The ingredients listed in Table D below were placed in amber glass bottles and mixed well. The formulated, liquid coating systems were coated onto phosphatized steel panels with a No. 20 wire-wound rod and cured an air-circulating oven for 30 minutes at 110°C. A second set of panels were prepared and cured for 15 minutes at 120°C. The cured coatings, which were of high gloss and tack free, were cooled to room temperature and tested for the properties given in Table D.

Table D

| Ingredients | Example 8 Grams | Example 8 Wt. Percent | Example 9 Grams | Example 9 Wt. Percent |
|---|---|---|---|---|
| Example 5 Polyester | 6.48 | 25.92 | 3.81 | 15.24 |
| Epoxide I | 18.27 | 73.08 | 18.78 | 75.12 |
| Vinyl Ester I | --- | --- | 2.16 | 8.64 |
| Surfactant II | 0.125 | 0.50 | 0.125 | 0.50 |
| Catalyst II | 0.125 | 0.50 | 0.125 | 0.50 |
| Properties after cure at 110°C for 30 min. | | | | |
| Double acetone rubs | 100 (1) | | 100 (1) | |
| Pencil Hardness | 3H | | >6H | |
| Crosshatch Adhesion, % | 100 | | 100 | |
| Properties after cure at 120°C for 15 min. | | | | |
| Double acetone rubs | 100 (1) | | 100 (1) | |
| Pencil Hardness | 2H | | 4H-5H | |
| Crosshatch Adhesion, % | 100 | | 100 | |

In addition to these properties, when the coatings were contacted with pH 2.0 sulfuric acid solution at 50 ° C and 70 ° C, they exhibited excellent acid resistance indicating that they would have good resistance to hostile environments such as those encountered in acid rain situations. Thus, very hard coatings with excellent solvent resistance and adhesion were formed at low temperatures when the polyesters of this invention were reacted with a cycloaliphatic epoxide in the presence of a triflic acid salt.

Examples 10 and 11

Polyesters were prepared in the same manner as that of Example 5 except the ingredients listed in Table E below were used. In addition, a small amount of xylene was used as an inert solvent for the reaction. The polyesters had the properties given in Table E.

Table E

| Ingredients | Example 10 Grams | Example 10 Moles | Example 11 Grams | Example 11 Moles |
|---|---|---|---|---|
| 2-Ethyl-3-propyl-1,5-pentanediol | 125.0 | 0.72 | 125.0 | 0.72 |
| Trimethylolpropane | 8.8 | 0.07 | 8.8 | 0.07 |
| Adipic acid | 29.6 | 0.20 | 14.7 | 0.10 |
| Isophthalic acid | 63.8 | 0.38 | 66.7 | 0.40 |
| Dibutyltin oxide catalyst | 0.43 | —— | 0.43 | —— |
| Xylene | 2.3 | —— | 2.2 | —— |
| Polyester Properties | | | | |
| Hydroxyl Number | 72.8 | —— | 76.7 | —— |
| Acid Number | 4.33 | —— | 3.05 | —— |
| Gel Permeation Molecular Weight | | | | |
| Number Average | 1316 | —— | 1026 | —— |
| Weight Average | 2789 | —— | 1791 | —— |
| Polydispersity | 2.12 | —— | 1.75 | —— |
| Brookfield Viscosity, cP, 25°C | 462,000 | —— | 169,500 | —— |

Examples 12 and 13

These examples describe preparation of thermally curable coatings prepared from the polyesters prepared in Examples 10 and 11, an aminoplast crosslinking agent and other ingredients listed in Table F below and cured in the same manner as described in Example 7. The coatings were then tested by the indicated tests in Table F and the results are given in Table F.

Table F

| Ingredients, grams | Example 12 | Example 13 |
|---|---|---|
| Example 10 Polyester | 26.3 | ---- |
| Example 11 Polyester | ---- | 26.3 |
| Melamine I | 11.5 | 11.5 |
| Methyl amyl ketone | 12.5 | 12.5 |
| Surfactant I | 0.8 | 0.8 |
| Surfactant II | 0.8 | 0.8 |
| Catalyst I | 0.3 | 0.3 |
| **Cured Film Properties** | | |
| Cured film thickness, mils | 1.9 | 1.8 |
| Specular Gloss (20°) (ASTM D 523-78) | 90.6 | 93.7 |
| Specular Gloss (60°) (ASTM D 523-78) | 97.8 | 98.9 |
| Distinctness of Reflected Image | 57 | 51 |
| Pencil Hardness (ASTM D 3363-74) | 2H | 3H |
| Impact Resistance (ASTM D 2794-84) | | |
| Forward, in-lbs | >300 | 275 |
| Reverse, in-lbs | >300 | 200 |
| Gloss Retention, 20°, % | 99.9 | 99.5 |
| Methyl ethyl ketone double rubs | >500 | >500 |
| Crosshatch Adhesion | 100% | 100% |
| Conical Mandril Bend | Passed | Passed |

The coatings of Examples 12 and 13 have an excellent combination of high gloss, distinctness of reflected image, hardness, both forward and reverse impact resistance, flexibility, adhesion, solvent resistance, and gloss retention when immersed in hot water.

Example 14

A polyester product was prepared by charging 75.2 grams (0.43 mole) of 2-ethyl-3-propyl-1,5-pentanediol, 3.5 grams (0.026 mole) of trimethylolpropane, 16.2 grams (0.10 mole) of isophthalic acid, 14.3 grams (0.10 mole) of adipic acid, and 0.22 grams (0.2 weight percent) dibutyltin oxide catalyst to a four-necked, one-liter round bottom flask equipped with a mechanical stirrer, thermometer, nitrogen sparging tube, and a simple distillation head. During the reaction, the ingredients were kept under a nitrogen sparge with stirring. The system was gradually heated to 160°C over a one-hour period. The temperature was maintained at 160°C with a Therm-O-Watch controller for one hour, and then the temperature was increased in 10 degree increments every 45 minutes until a reaction temperature of 220°C was attained. This temperature was maintained for 30 minutes after which the system was allowed to cool to room temperature. All water of condensation formed by the reaction was removed with the simple distillation head. The resulting polyester had a Brookfield viscosity of 5,440 centipoise at 23°C, a hydroxyl number of 258, and an acid number of 2.23. Molecular weight determination by gel permeation chromatography using a polystyrene standard resulted in an apparent number average molecular weight of 831, a weight-average molecular weight of 1161, and a polydispersity of 1.40.

A composition containing 3.75 grams of the polyester product, 18.5 grams of Epoxide I, 2.125 grams of Vinyl Ester II, 0.125 grams of Surfactant III, and 0.50 grams of Photoinitiator I was placed in an amber

bottle, well mixed and cured on phosphatized steel and on cleaned Y test pattern boards as described below.

A portion of the composition was coated onto Bonderite 37 steel with a No. 20 wire-wound rod, cured with one pass under a 300 watt-per-inch medium pressure mercury vapor lamp (American Ultraviolet Company equipment) at 30 feet-per-minute to form a cured coating.

Another portion of the composition was coated onto a Y test pattern printed circuit board by first cleaning the board with a detergent solution and a soft brush, rinsing the board several times with distilled water while lightly scrubbing with a soft brush, and then rinsing with acetone. The board was dried in a circulating-air oven at 65°C for 30 minutes. The resistance gap between the Y pattern was coated by pouring the coating composition over just the Y pattern of the Y test pattern board (i.e., most of the board was not coated) and cured with one pass under a 300 watt-per-inch medium pressure mercury vapor lamp (American Ultraviolet Company equipment) at 30 feet-per-minute to form a cured coating. After ultraviolet light exposure, the insulation resistance of the cured coating was measured at various times while remaining at room temperature (67°C) and humidity conditions which were high but not measured and which varied from day to day.

The cured coating passed 100 acetone double rubs, had 100 percent crosshatch adhesion, and a 2H hardness on the steel substrate. On the Y-pattern test board the insulation resistance was $40 \times 10^{12}$ ohms.

Although the invention has been illustrated by certain of the preceding examples, it is not to be construed as being limited thereby; but rather, the invention encompasses the generic area as hereinbefore disclosed. Various modifications and embodiments can be made without departing from the spirit and scope thereof.

## Related Applications

The following are related, commonly assigned applications, filed on an even date herewith:

U.S. Patent Application Serial No. 790,875 (D-16840); U.S. Patent Application Serial No. 790,873 (D-16842); U.S. Patent Application Serial No. 790,896 (D-16843); U.S. Patent Application Serial No. 790,874 (D-16844); and U.S. Patent Application Serial No. 790,895 (D-16845); all of which are incorporated herein by reference.

## Claims

**1.** A liquid hydrocarbon diol represented by the formula:

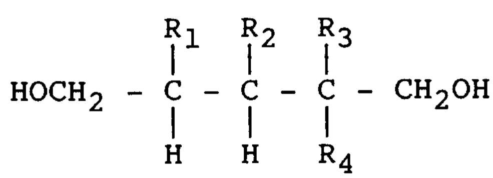

wherein $R_1$ is hydrogen or linear or branched alkyl having from 1 to 3 carbon atoms, and $R_2$, $R_3$ and $R_4$ are the same or different and are hydrogen or linear or branched alkyl having from 1 to 4 carbon atoms, said liquid hydrocarbon diol existing as a liquid at a temperature of 35°C or less; provided (a) at least 2 of $R_1$, $R_2$, $R_3$ and $R_4$ are other than hydrogen; (b) the total number of carbon atoms in $R_1$, $R_2$, $R_3$ and $R_4$ together is 5 or more except as follows: (i) when $R_2$ is ethyl and one of $R_1$, $R_3$ and $R_4$ is methyl, then the remaining 2 of $R_1$, $R_3$ and $R_4$ can be hydrogen; (ii) when $R_3$ and $R_4$ are independently methyl and propyl, then $R_1$ and $R_2$ can be hydrogen; and (iii) when $R_1$ is methyl and $R_2$ is ethyl and one of $R_3$ and $R_4$ is methyl, then the remaining $R_3$ or $R_4$ can be hydrogen; (c) when the total number of carbon atoms in $R_1$ and $R_2$ is 4 or more, then the total number of carbon atoms in $R_2$ is 3 or less except when $R_1$ is propyl and $R_3$ and $R_4$ are hydrogen, then $R_2$ can be butyl; and (d) when $R_1$ is ethyl and $R_2$ is propyl, then $R_3$ is other than ethyl.

**2.** The liquid hydrocarbon diol of claim 1 which is selected from 3-ethyl-2-methyl-1,5-pentanediol, 2-ethyl-3-propyl-1,5-pentanediol, 2,4-dimethyl-3-ethyl-1,5-pentanediol, 2-ethyl-4-methyl-3-propyl-1,5-pentanediol, 2,3-diethyl-4-methyl-1,5-pentanediol, 3-ethyl-2,2,4-trimethyl-1,5-pentanediol, 2,2-dimethyl-4-ethyl-3-propyl-1,5-pentanediol, 2-methyl-2-propyl-1,5-pentanediol, 2,4-dimethyl-3-ethyl-2-propyl-1,5-pentanediol, 2,3-dipropyl-4-ethyl – 2 – methyl – 1,5 – pentanediol, 2 – butyl – 2 – ethyl – 1,5 – pentanediol, 2 – butyl – 2,3 – diethyl – 4 – methyl – 1,5 – pentanediol, 2 – butyl – 2,4 – diethyl – 3 – propyl – 1,5 – pentanediol and 3 – butyl – 2 – propyl – 1,5 – pentanediol.

**3.** A process comprising:

a) contacting a substituted vinyl ether with a substituted or unsubstituted acrolein to form a substituted 3,4 – dihydropyran;

b) contacting the substituted 3,4 – dihydropyran with an acid catalyst to form a substituted dialdehyde; and

c) hydrogenating the substituted dialdehyde in the presence of a catalyst to form a liquid hydrocarbon diol represented by the formula:

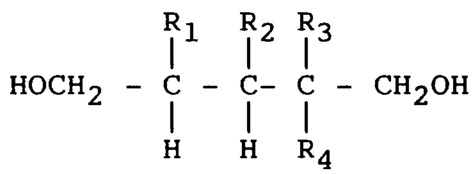

wherein $R_1$ is hydrogen or linear or branched alkyl having from 1 to 3 carbon atoms, and $R_2$, $R_3$ and $R_4$ are the same or different and are hydrogen or linear or branched alkyl having from 1 to 4 carbon atoms, said liquid hydrocarbon diol existing as a liquid at a temperature of 35 ° C or less; provided (a) at least 2 of $R_1$, $R_2$, $R_3$ and $R_4$ are other than hydrogen; (b) the total number of carbon atoms in $R_1$, $R_2$, $R_3$ and $R_4$ together is 5 or more except as follows: (i) when $R_2$ is ethyl and one of $R_1$, $R_3$ and $R_4$ is methyl, then the remaining 2 of $R_1$, $R_3$ and $R_4$ can be hydrogen; (ii) when $R_3$ and $R_4$ are independently methyl and propyl, then $R_1$ and $R_2$ can be hydrogen; and (iii) when $R_1$ is methyl and $R_2$ is ethyl and one of $R_3$ and $R_4$ is methyl, then the remaining $R_3$ or $R_4$ can be hydrogen; (c) when the total number of carbon atoms in $R_1$ and $R_2$ is 4 or more, then the total number of carbon atoms is $R_2$ is 3 or less except when $R_1$ is propyl and $R_3$ and $R_4$ are hydrogen, then $R_2$ can be butyl; and (d) when $R_1$ is ethyl and $R_2$ is propyl, then $R_3$ is other than ethyl.

**4.** The process of claim 3 wherein the substituted vinyl ether is methyl vinyl ether or ethyl vinyl ether, the substituted acrolein is 2 – ethyl – 2 – hexenal, and the substituted 3,4 – dihydropyran is selected from 2 – alkoxy – 5 – ethyl – 4 – propyl – 3,4 – dihydro – 1,2 – pyran, 2 – ethoxy – 5 – ethyl – 4 – propyl – 3,4 – dihydro – 1,2 – pyran, 2 – methoxy – 5 – ethyl – 4 – propyl – 3,4 – dihydro – 1,2 – pyran, 2 – alkoxy – 4 – ethyl – 5 – methyl – 3,4 – dihydro – 1,2 – pyran, 2 – alkoxy – 4 – ethyl – 3,5 – dimethyl – 3,4 – dihydro – 1,2 – pyran, 2 – alkoxy – 5 – ethyl – 3 – methyl – 4 – propyl – 3,4 – dihydro – 1,2 – pyran, 2 – alkoxy – 3,4 – diethyl – 5 – methyl – 3,4 – dihydro – 1,2 – pyran, 2 – alkoxy – 4 – ethyl – 3,3',5 – trimethyl – 3,4 – dihydro – 1,2 – pyran, 2 – alkoxy – 3,3' – dimethyl – 4 – propyl – 3,4 – dihydro – 1,2 – pyran, 2 – alkoxy – 3 – methyl – 3' – propyl – 3,4 – dihydro – 1,2 – pyran, 2 – alkoxy – 4 – ethyl – 5 – methyl – 3 – methyl – 3' – propyl – 3,4 – dihydro – 1,2 – pyran, 2 – alkoxy – 5 – ethyl – 3 – methyl – 3',4 – dipropyl – 3,4 – dihydro – 1,2 – pyran, 2 – alkoxy – 3 – butyl – 3' – ethyl – 3,4 – dihydro – 1,2 – pyran, 2 – alkoxy – 3 – butyl – 3',4 – diethyl – 5 – methyl – 3,4 – dihydro – 1,2 – pyran, and 2 – alkoxy – 3 – butyl – 3',5 – diethyl – 4 – propyl – 3,4 – dihydro – 1,2 – pyran, wherein alkoxy is methoxy, ethoxy, n – propoxy, isopropoxy, n – butoxy, i – butoxy, s – butoxy and t – butoxy.

**5.** The process of claim 3 in which the substituted dialdehyde is selected from 3 – ethyl – 2 – methyl – 1,5 – pentanedial, 2 – ethyl – 3 – propyl – 1,5 – pentanedial, 3 – ethyl – 2,4 – dimethyl – 1,5 – pentanedial, 2 – ethyl – 4 – methyl – 3 – propyl – 1,5 – pentanedial, 3,4 – diethyl – 2 – methyl – 1,5 – pentanedial, 3 – ethyl – 2,4,4' – trimethyl – 1,5 – pentanedial, 2 – ethyl – 4,4' – dimethyl – 3 – propyl – 1,5 – pentanedial, 2 – methyl – 2' – propyl – 1,5 – pentanedial, 3 – ethyl – 2,4 – dimethyl – 4' – propyl – 1,5 – pentanedial, 2 – ethyl – 4 – methyl – 3,4' – dipropyl – 1,5 – pentanedial, 2 – butyl – 2' – ethyl – 1,5 – pentanedial, 4 – butyl – 3,4 – diethyl – 2 – methyl – 1,5 – pentanedial, and 4 – butyl – 2,4' – diethyl – 3 – propyl – 1,5 – pentanedial.

**6.** The process of claim 3 in which the liquid hydrocarbon diol is selected from 3 – ethyl – 2 – methyl – 1,5 – pentanediol, 2 – ethyl – 3 – propyl – 1,5 – pentanediol, 2,4 – dimethyl – 3 – ethyl – 1,5 – pentanediol, 2 – ethyl – 4 – methyl – 3 – propyl – 1,5 – pentanediol, 2,3 – diethyl – 4 – methyl – 1,5 – pentanediol, 3 – ethyl – 2,2,4 – trimethyl – 1,5 – pentanediol, 2,2 – dimethyl – 4 – ethyl – 3 – propyl – 1,5 – pentanediol, 2 –

methyl-2-propyl-1,5-pentanediol, 2,4-dimethyl-3-ethyl-2-propyl-1,5-pentanediol, 2,3-dipropyl-4-ethyl-2-methyl-1,5-pentanediol, 2-butyl-2-ethyl-1,5-pentanediol, 2-butyl-2,3-diethyl-4-methyl-1,5-pentanediol, 2-butyl-2,4-diethyl-3-propyl-1,5-pentanediol and 3-butyl-2-propyl-1,5-pentanediol.

**7.** The process of claim 3 further comprising derivatizing the liquid hydrocarbon diol.

**8.** The process of claim 7 in which the derivatizing reaction comprises an oxidation, alkoxylation, carboxylation, reduction, hydrogenation, dehydrogenation, condensation, amination, esterification, etherification, silylation, alkylation or acylation reaction.

**9.** The product produced by step (a) of the process of claim 3, step (b) of the process of claim 3, step (c) of the process of claim 3 or the process of claim 7.

**10.** A derivative of the product of claim 9 or the liquid hydrocarbon diol of claim 1.